# EUROPEAN PATENT SPECIFICATION

(11) **EP 3 393 600 B1**
(45) Date of publication and mention of the grant of the patent: **21.02.2024**
(21) Application number: 16774719.5
(22) Date of filing: 26.08.2016
(51) Int. Cl.: A61Q 17/04, A61Q 19/00, A61K 8/06, A61K 8/37, A61K 8/39, A61K 8/86, A61K 8/46, A61K 8/49

(54) **SUN CARE COMPOSITION BASED ON SMALL PARTICLE O/W EMULSION**
SONNENSCHUTZZUSAMMENSETZUNG AUF DER BASIS EINER O/W-EMULSION AUS KLEINEN PARTIKELN
COMPOSITION DE PROTECTION SOLAIRE À BASE D'UNE ÉMULSION D'HUILE DANS L'EAU DE PETITES PARTICULES

(30) Priority: 22.12.2015 JP 2015250446
(43) Date of publication of application: 31.10.2018
(73) Proprietor: L'OREAL, 75008 Paris (FR); Sinha, Ritesh, Takatsu-ku Kawasaki-shi Kanagawa 213-0012 (JP); Alam, Mohammad Mydul, Takatsu-ku Kawasaki-shi Kanagawa 213-0012 (JP); Koike, Toru, Kanagawa 213-0012 (JP)
(72) Inventor: SINHA, Ritesh, Kawasaki-shi Kanagawa 213-0012 (JP); ALAM, Mohammad, Mydul, Kawasaki-shi Kanagawa 213-0012 (JP); KOIKE, Toru, Kawasaki-shi Kanagawa 213-0012 (JP)
(74) Representative: Casalonga
(86) International application number: PCT/JP2016/075742
(87) International publication number: WO 2017/110153

(56) References cited:
- EP-A2- 1 092 414
- WO-A1-2014/098264
- WO-A2-01/10390
- DE-A1- 10 214 059
- US-A1- 2006 165 616
- DATABASE GNPD [Online] MINTEL; 1 June 2013 (2013-06-01), "Diamond Peptide Whitening Cream SPF 15", XP002763474, Database accession no. 2091271
- CHOOL BOO YONG ED - MANN GIOVANNI E ET AL: "Scutellaria Radix Extract as a Natural Cosmetic Ingredient for Protection of Human Skin Against UV Radiation", FREE RADICAL BIOLOGY AND MEDICINE, vol. 87, 182, 1 October 2015 (2015-10-01), pages 1-1, XP029334113, ISSN: 0891-5849, DOI: 10.1016/J.FREERADBIOMED.2015.10.226
- MASON T G ET AL: "TOPICAL REVIEW; Nanoemulsions: formation, structure, and physical properties", JOURNAL OF PHYSICS: CONDENSED MATTER, vol. 18, no. 41, 18 October 2006 (2006-10-18), pages R635-R666, XP020102622, INSTITUTE OF PHYSICS PUBLISHING, BRISTOL, GB ISSN: 0953-8984, DOI: 10.1088/0953-8984/18/41/R01
- ZYDUS WELLNESS: "Diamond Peptide Whitening Cream SPF 15", GNPD, MINTEL, 1 June 2013 (2013-06-01), XP002763474,

## Description

### TECHNICAL FIELD

The present invention relates to a sun care composition, in particular a sun care cosmetic composition in the form of an oil-in-water (O/W) emulsion, for a keratin substance such as skin.

### BACKGROUND ART

It is known practice, in the cosmetics or dermatological field, to use O/W emulsions. These emulsions which consist of oil phases dispersed in an aqueous phase have an external aqueous phase, and therefore cosmetic/dermatological products based on the O/W emulsions are pleasant to use due to the feeling of freshness that the external aqueous phase can provide.

Owing to this benefit of the O/W emulsions, they also can be used in sun care cosmetic products for the skin, as known e.g. from EP1092414A2 or DE10214059A1.

For sun care products, good UV protecting effects are among the key functions.

### DISCLOSURE OF INVENTION

An objective of the present invention is to provide a composition, preferably a cosmetic composition, in the form of an O/W emulsion for a keratin substance, such as skin, which can provide improved UV protecting effects.

The above objective of the present invention can be achieved by a composition in the form of an O/W emulsion having oil phases dispersed in an aqueous phase, comprising:
(a) at least one oil;
(b) at least two hydrophilic UV filters comprising at least one hydrophilic UV-A filter and at least one hydrophilic UV-B filter;
(c) water; and
(d) emulsifiers comprising at least one nonionic emulsifier;

wherein the number-average diameter of the oil phase droplets is less than 200 nm, and the amount of the oil is from 0.5 to 5% by weight relative to the total weight of the composition; the hydrophilic UV-A filter is selected from terephthalylidene dicamphor sulfonic acid and salts thereof, bisbenzoxazolyl derivatives chosen from disodium phenyl dibenzimidazole tetrasulfonate and salts thereof;
the hydrophilic UV-B filter is selected from p-aminobenzoic (PABA) derivatives chosen from PABA, Glyceryl PABA and PEG-25 PABA and salts thereof, phenylbenzimidazole sulfonic acid and salts thereof, ferulic acid and salts thereof, salicylic acid and salts thereof, DEA methoxycinnamate and salts thereof, benzylidene camphor sulfonic acid and salts thereof, and camphor benzalkonium methosulfate and salts thereof; and
the amount of the (b) hydrophilic UV filters is from 5 to 25% by weight relative to the total weight of the composition.

The number-average diameter of the oil phase droplets may be less than 180 nm, preferably less than 160 nm, and more preferably less than 140 nm.

The number-average diameter of the oil phase droplets may be more than 1 nm, more preferably more than 5 nm, and even more preferably more than 10 nm, and in particular more than 20 nm.

The oil may be selected from ester oils, preferably having a molecular weight less than 600 g/mol such as isopropyl myristate, isopropyl palmitate, ethyl hexyl palmitate; artificial triglycerides such as capryl caprylyl glycerides; hydrocarbon oil; and mineral oil such as paraffine oil, and mixtures thereof.

The amount of the oil may be from 1 to 5 % by weight, relative to the total weight of the composition.

The hydrophilic UV filter may be selected from phenylbenzimidazole sulfonic acid, terephthalylidene dicamphor sulfonic acid, and mixtures thereof.

The amount of the water in the composition may be from 50 to 99% by weight, preferably from 55 to 95% by weight, and more preferably 60 to 90% by weight, relative to the total weight of the composition.

The composition may comprise at least one hydrophobic UV filter as defined below, preferably in an amount of from 0.1 to 10% by weight, and more preferably from 0.2 to 5% by weight, relative to the total weight of the composition.

The composition may further comprise at least one gelling agent, preferably in an amount of from 0.1 to 20% by weight, preferably from 0.2 to 15% by weight, and more preferably from 1 to 10% by weight, relative to the total weight of the composition.

The nonionic emulsifier is preferably selected from fatty acids, fatty alcohols, polyethoxylated fatty acids, polyglycerolated fatty acids and mixture thereof.

The amount of the emulsifier may be from 0.1 to 30% by weight, preferably from 0.5 to 20% by weight, and more preferably from 1 to 10% by weight, relative to the total weight of the composition.

The composition may be a cosmetic composition, preferably a cosmetic composition for a keratin substance, and more preferably a skin cosmetic composition.

The present invention also relates to a composition according to the present invention, for use in a process for protecting a keratin substance from ultraviolet radiation, which is applied onto the keratin substance.

### BEST MODE FOR CARRYING OUT THE INVENTION

After diligent research, the inventors have found that an introduction of a nano-sized oil phase into aqueous phase containing hydrophilic UV filer can surprisingly provide compositions in the form of an O/W emulsion with improved UV protecting effect.

Thus, the composition in the form of an O/W emulsion having oil phases dispersed in an aqueous phase, preferably a cosmetic composition for a keratin substance, preferably skin, according to the present invention comprises:
(a) at least one oil;
(b) at least two hydrophilic UV filters comprising at least one hydrophilic UV-A filter and at least one hydrophilic UV-B filter;
(c) water; and
(d) emulsifiers comprising at least one nonionic emulsifier;

wherein the number-average diameter of the oil phase droplets is less than 200 nm, and the amount of the oil is from 0.5 to 5% by weight relative to the total weight of the composition; the hydrophilic UV-A filter is selected from terephthalylidene dicamphor sulfonic acid and salts thereof, bisbenzoxazolyl derivatives chosen from disodium phenyl dibenzimidazole tetrasulfonate and salts thereof;
the hydrophilic UV-B filter is selected from p-aminobenzoic (PABA) derivatives chosen from PABA, Glyceryl PABA and PEG-25 PABA and salts thereof, phenylbenzimidazole sulfonic acid and salts thereof, ferulic acid and salts thereof, salicylic acid and salts thereof, DEA methoxycinnamate and salts thereof, benzylidene camphor sulfonic acid and salts thereof, and camphor benzalkonium methosulfate and salts thereof; and
the amount of the (b) hydrophilic UV filters is from 5 to 25% by weight relative to the total weight of the composition.

The composition according to the present invention can exhibit improved UV protecting effects.

Hereafter, the composition according to the present invention will be described in a detailed manner.

### [Composition]

The composition according to the present invention is in the form of an O/W emulsion having oil phases dispersed in an aqueous phase, wherein the oil phase forms a nano-sized droplets having the number-average diameter of less than 200 nm. The composition is as defined above in the present specification.

It is preferable that the composition according to the present invention is a cosmetic composition, in particular a cosmetic sun care composition for a keratin substance such as skin, i.e. skin cosmetic composition.

The composition according to the present invention can exhibit improved UV protecting effects. Furthermore, the composition according to the present invention can have a transparent or translucent appearance due to a small size of the oil phase, which is preferable in use for cosmetic topical compositions for a keratin substance, such as skin.

### <Oil Phase>

The oil phase of the O/W emulsion according to the present invention includes at least one (a) oil. The amount of the oil phase can range from 0.1 to 80% by weight, preferably from 0.2 to 60% by weight, and more preferably from 0.5 to 30% by weight, relative to the total weight of the composition.

The oil phase is in the form of a nano-sized droplet. The number-average diameter of the oil phase, i.e. oil droplets, is less than 200 nm, preferably less than 180 nm, and more preferably less than 160 nm, and in particular less than 140 nm. In general, the number-average diameter of the oil phase droplets is preferably more than 1 nm, more preferably more than 5 nm, and even more preferably more than 10 nm, and in particular more than 20 nm. The size of the oil droplet can be measured by, for example, Particle size analyzer (Vasco, Cordouan Technologies).

One assumable hypothesis of the present invention being capable to provide improved UV protecting effects is that the nano-sized droplet has a tendency to spread the composition homogeneously onto the surface of a keratinous substance, which can provide a homogeneous and thick deposition of the UV filter included in the composition on the surface of the keratinous substance.

### (Oil)

The composition according to the present invention comprises (a) at least one oil. Two or more (a) oils may be used in combination. Thus, a single type of oil or a combination of different types of oil may be used.

As used herein, the expression "oil" means a fatty compound or substance which is in the form of a liquid or a paste (non-solid) at room temperature (25°C) under atmospheric pressure (760 mmHg). These oil(s) may be volatile or non-volatile, preferably non-volatile.

The (a) oil may be a non-polar oil such as a hydrocarbon oil, a silicone oil, or the like; a polar oil such as a plant or animal oil and an ester oil or an ether oil; or a mixture thereof.

It is preferable that the (a) oil be selected from the group consisting of oils of plant or animal origin, synthetic oils, silicone oils, and hydrocarbon oils, and mixtures thereof.

As examples of plant oils, mention may be made of, for example, linseed oil, camellia oil, macadamia nut oil, corn oil, mink oil, olive oil, avocado oil, sasanqua oil, castor oil, safflower oil, jojoba oil, sunflower oil, almond oil, rapeseed oil, sesame oil, soybean oil, peanut oil, and mixtures thereof.

As examples of animal oils, mention may be made of, for example, squalene and squalane.

As examples of synthetic oils, mention may be made of alkane oils such as isododecane and isohexadecane, ester oils, ether oils, and artificial triglycerides.

The ester oils are preferably liquid esters of saturated or unsaturated, linear or branched C₁-C₂₆ aliphatic monoacids or polyacids and of saturated or unsaturated, linear or branched C₁-C₂₆ aliphatic monoalcohols or polyalcohols, the total number of carbon atoms of the esters being preferably greater than or equal to 10, and preferably smaller than or equal to 30.

Preferably, for the esters of monoalcohols, at least one from among the alcohol and the acid from which the esters of the invention are derived is branched.

Among the monoesters of monoacids and of monoalcohols, mention may be made of ethyl palmitate, ethyl hexyl palmitate, isopropyl palmitate, dicaprylyl carbonate, alkyl myristates such as isopropyl myristate or ethyl myristate, isocetyl stearate, 2-ethylhexyl isononanoate, isononyl isononanoate, isodecyl neopentanoate and isostearyl neopentanoate.

Esters of C₄-C₂₂ dicarboxylic or tricarboxylic acids and of C₁-C₂₂ alcohols and esters of monocarboxylic, dicarboxylic or tricarboxylic acids and of non-sugar C₄-C₂₆ dihydroxy, trihydroxy, tetrahydroxy or pentahydroxy alcohols may also be used.

Mention may especially be made of: diethyl sebacate; isopropyl lauroyl sarcosinate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

As ester oils, one can use sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides or polysaccharides.

Examples of suitable sugars that may be mentioned include sucrose (or saccharose), glucose, galactose, ribose, fucose, maltose, fructose, mannose, arabinose, xylose and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may have one to three conjugated or non-conjugated carbon-carbon double bonds.

The esters according to this variant may also be selected from monoesters, diesters, triesters, tetraesters and polyesters, and mixtures thereof.

These esters may be, for example, oleates, laurates, palmitates, myristates, behenates, cocoates, stearates, linoleates, linolenates, caprates and arachidonates, or mixtures thereof such as, especially, oleopalmitate, oleostearate and palmitostearate mixed esters, as well as pentaerythrityl tetraethyl hexanoate.

More particularly, use is made of monoesters and diesters and especially sucrose, glucose or methylglucose monooleates or dioleates, stearates, behenates, oleopalmitates, linoleates, linolenates and oleostearates.

An example that may be mentioned is the product sold under the name Glucate^{®} DO by the company Amerchol, which is a methylglucose dioleate.

As examples of preferable ester oils, mention may be made of, for example, diisopropyl adipate, dioctyl adipate, 2-ethylhexyl hexanoate, ethyl laurate, cetyl octanoate, octyldodecyl octanoate, isodecyl neopentanoate, myristyl propionate, 2-ethylhexyl 2-ethylhexanoate, 2-ethylhexyl octanoate, 2-ethylhexyl caprylate/caprate, methyl palmitate, ethyl palmitate, isopropyl palmitate, dicaprylyl carbonate, isopropyl lauroyl sarcosinate, isononyl isononanoate, ethylhexyl palmitate, isohexyl laurate, hexyl laurate, isocetyl stearate, isopropyl isostearate, isopropyl myristate, isodecyl oleate, glyceryl tri(2-ethylhexanoate), pentaerythrithyl tetra(2-ethylhexanoate), 2-ethylhexyl succinate, diethyl sebacate, and mixtures thereof.

As examples of artificial triglycerides, mention may be made of, for example, capryl caprylyl glycerides, glyceryl trimyristate, glyceryl tripalmitate, glyceryl trilinolenate, glyceryl trilaurate, glyceryl tricaprate, glyceryl tricaprylate, glyceryl tri(caprate/caprylate) and glyceryl tri(caprate/caprylate/linolenate).

As examples of silicone oils, mention may be made of, for example, linear organopolysiloxanes such as dimethylpolysiloxane, methylphenylpolysiloxane, methylhydrogenpolysiloxane, and the like; cyclic organopolysiloxanes such as octamethylcyclotetrasiloxane, decamethylcyclopentasiloxane, dodecamethylcyclohexasiloxane, and the like; and mixtures thereof.

Preferably, silicone oil is chosen from liquid polydialkylsiloxanes, especially liquid polydimethylsiloxanes (PDMS) and liquid polyorganosiloxanes comprising at least one aryl group.

These silicone oils may also be organomodified. The organomodified silicones that can be used in accordance with the present invention are silicone oils as defined above and comprising in their structure one or more organofunctional groups attached via a hydrocarbon-based group.

Organopolysiloxanes are defined in greater detail in Walter Noll's Chemistry and Technology of Silicones (1968), Academic Press. They may be volatile or non-volatile.

When they are volatile, the silicones are more particularly chosen from those having a boiling point of between 60°C and 260°C, and even more particularly from:
(i) cyclic polydialkylsiloxanes comprising from 3 to 7 and preferably 4 to 5 silicon atoms. These are, for example, octamethylcyclotetrasiloxane sold in particular under the name Volatile Silicone^{®} 7207 by Union Carbide or Silbione^{®} 70045 V2 by Rhodia, decamethylcyclopentasiloxane sold under the name Volatile Silicone^{®} 7158 by Union Carbide, Silbione^{®} 70045 V5 by Rhodia, and dodecamethylcyclopentasiloxane sold under the name Silsoft 1217 by Momentive Performance Materials, and mixtures thereof. Mention may also be made of cyclocopolymers of the type such as dimethylsiloxane/methylalkylsiloxane, such as Silicone Volatile^{®} FZ 3109 sold by the company Union Carbide, of formula: 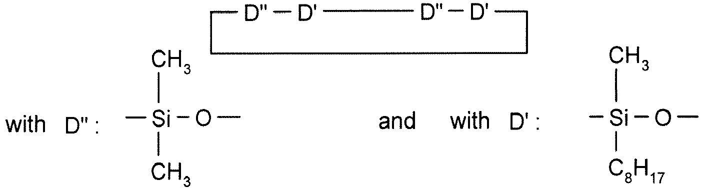 Mention may also be made of mixtures of cyclic polydialkylsiloxanes with organosilicon compounds, such as the mixture of octamethylcyclotetrasiloxane and tetratrimethylsilylpentaerythritol (50/50) and the mixture of octamethylcyclotetrasiloxane and oxy-1,1'-bis(2,2,2',2',3,3'-hexatrimethylsilyloxy)neopentane;
(ii) linear volatile polydialkylsiloxanes containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, Volatile Silicone Fluids for Cosmetics. The viscosity of the silicones is measured at 25°C according to ASTM standard 445 Appendix C.

Non-volatile polydialkylsiloxanes may also be used. These non-volatile silicones are more particularly chosen from polydialkylsiloxanes, among which mention may be made mainly of polydimethylsiloxanes containing trimethylsilyl end groups.

Among these polydialkylsiloxanes, mention may be made, in a non-limiting manner, of the following commercial products:
- the Silbione^{®} oils of the 47 and 70 047 series or the Mirasil^{®} oils sold by Rhodia, for instance the oil 70 047 V 500 000;
- the oils of the Mirasil^{®} series sold by the company Rhodia;
- the oils of the 200 series from the company Dow Coming, such as DC200 with a viscosity of 60 000 mm²/s;
- the Viscasil^{®} oils from General Electric and certain oils of the SF series (SF 96, SF 18) from General Electric.

Mention may also be made of polydimethylsiloxanes containing dimethylsilanol end groups known under the name dimethiconol (CTFA), such as the oils of the 48 series from the company Rhodia.

Among the silicones containing aryl groups are polydiarylsiloxanes, especially polydiphenylsiloxanes and polyalkylarylsiloxanes. Examples that may be mentioned include the products sold under the following names:
- the Silbione^{®} oils of the 70 641 series from Rhodia;
- the oils of the Rhodorsil^{®} 70 633 and 763 series from Rhodia;
- the oil Dow Corning 556 Cosmetic Grade Fluid from Dow Corning;
- the silicones of the PK series from Bayer, such as the product PK20;
- certain oils of the SF series from General Electric, such as SF 1023, SF 1154, SF 1250 and SF 1265.

The organomodified liquid silicones may especially contain polyethyleneoxy and/or polypropyleneoxy groups. Mention may thus be made of the silicone KF-6017 proposed by Shin-Etsu, and the oils Silwet^{®} L722 and L77 from the company Union Carbide.

Hydrocarbon oils may be chosen from:
- linear or branched, optionally cyclic, C₆-C₁₆ lower alkanes. Examples that may be mentioned include hexane, undecane, dodecane, tridecane, and isoparaffins, for instance isohexadecane, isododecane and isodecane; and
- linear or branched hydrocarbons containing more than 16 carbon atoms, such as liquid paraffins, liquid petroleum jelly, polydecenes and hydrogenated polyisobutenes such as Parleam^{®}, and squalane.

As preferable examples of hydrocarbon oils, mention may be made of, for example, linear or branched hydrocarbons such as isohexadecane, isododecane, squalane, mineral oil (e.g., liquid paraffin), paraffin, vaseline or petrolatum, naphthalenes, and the like; hydrogenated polyisobutene, isoeicosane, and decene/butene copolymer; and mixtures thereof.

It may be preferable that the (a) oil be chosen from hydrocarbon oils which are in the form of a liquid at a room temperature.

It may be also preferable that the (a) oil be chosen from oils with molecular weight below 600 g/mol.

Preferably, the (a) oil has a low molecular weight such as below 600 g/mol, more preferably below 500 g/mol, in particular below 400 g/mol, chosen among ester or ether oils with a short hydrocarbon chain or chains (C₁-C₁₈, e.g., isopropyl myristate, isopropyl palmitate, isononyl isononanoate, dicaprylyl carbonate, ethyl hexyl palmitate, dicaprylyl ether, and isopropyl lauroyl sarcosinate), hydrocarbon oils with a short alkyl chain or chains (C₁-C₁₈, e.g., isododecane, isohexadecane, and squalane), and short alcohol type oils such as octyldodecanol.

It is also preferable that the (a) oil be selected from the group consisting of hydrocarbon oils, esters of C₄-C₂₂ dicarboxylic or tricarboxylic acids and of C₁-C₂₂ alcohols, and esters of C₄-C₂₂ monocarboxylic, dicarboxylic or tricarboxylic acids and of non-sugar C₄-C₂₆ dihydroxy, C₄-C₁₅ trihydroxy, tetrahydroxy or pentahydroxy alcohols, and mixtures thereof.

Preferably, the (a) oil is selected from ester oils having a molecular weight less than 600 g/mol such as isopropyl myristate, isopropyl palmitate, ethyl hexyl palmitate; artificial triglycerides such as capryl caprylyl glycerides; hydrocarbon oil; and mineral oil such as paraffine oil, and mixtures thereof.

The amount in the composition according to the present invention of the (a) oil ranges from 0.5 to 5% by weight, in particular 1 to 5 % by weight, relative to the total weight of the composition.

### <Aqueous Phase>

The aqueous phase of the O/W emulsion according to the present invention includes at least one (b) hydrophilic UV filter(s), (c) water and at least one (d) emulsifier. The amount of the aqueous phase can range from 1 to 99% by weight, preferably from 10 to 90% by weight, and more preferably from 20 to 80% by weight, and even more preferably from 30 to 70% by weight relative to the total weight of the composition.

### (Hydrophilic UV Filter)

The composition according to the present invention comprises (b) at least two hydrophilic UV filters comprising at least one hydrophilic UV-A filter and at least one hydrophilic UV-B filter. Two or more (b) hydrophilic UV filters may be used in combination.

The term "UV" here means ultraviolet radiation and comprises the UV-B region (260-320 nm in wavelength) and the UV-A region (320-400 nm in wavelength). Therefore, a UV filter means any material which has filtering effects of ultraviolet radiation in the wavelength of UV-A and/or UV-B regions.

The term "hydrophilic UV filter" is understood to mean any agent which screens out UV radiation and which is capable of being completely dissolved in the molecular state in the aqueous phase of the emulsion or of being dispersed in the colloidal form (for example in the micelle form) in the aqueous phase of the O/W emulsion.

The hydrophilic UV filter(s) used for the present invention may be active in the UV-A and/or UV-B region, preferably in the both of UV-A and UV-B regions in alone or a combination. Therefore, the hydrophilic UV filter(s), which can be used according to the invention, includes a hydrophilic UV-A filter capable of absorbing UV radiation from 320 to 400 nm, a hydrophilic UV-B filter capable of absorbing UV radiation from 280 to 320 nm, and a hydrophilic UV-A and UV-B filter capable of absorbing UV radiation from 280 to 400 nm.

The hydrophilic UV-A filter is selected from:
Terephthalylidene Dicamphor Sulfonic Acid and salts thereof, such as manufactured under the name "Mexoryl SX" by Chimex,
Bisbenzoxazolyl derivatives, such as described in Patent, EP 669 323 and US 2,463,264, chosen from Disodium Phenyl Dibenzimidazole Tetrasulfonate and salts thereof, such as that sold under the trade name "Neo Heliopan AP" by Haarmann and Reimer.

The hydrophilic UV-B filter is selected from:
p-Aminobenzoic (PABA) derivatives chosen from PABA, Glyceryl PABA and PEG-25 PABA, and salts thereof such as sold under the name "Uvinul P25" by BASF,
Phenylbenzimidazole Sulfonic Acid and salts thereof, such as sold in particular under the trade name "Eusolex 232" by Merck,
Ferulic Acid and salts thereof,
Salicylic Acid and salts thereof,
DEA methoxycinnamate and salts thereof,
Benzylidene Camphor Sulfonic Acid and salts thereof, such as manufactured under the name "Mexoryl SL" by Chimex,
Camphor Benzalkonium Methosulfate and salts thereof, such as manufactured under the name "Mexoryl SO" by Chimex.

The hydrophilic UV-A and UV-B filter includes, but is not limited to:
Benzophenone-4 and salts thereof, such as sold under the trade name "Uvinul MS40" by BASF,
Benzophenone-5 and salts thereof, and
Benzophenone-9 and salts thereof.

The salts of the compounds that may be used are chosen in particular from salts of alkali metals, for example sodium or potassium; salts of alkaline-earth metals, for example calcium, magnesium or strontium; metal salts, for example zinc, aluminium, manganese or copper; salts of ammonium of formula NH⁴⁺; quaternary ammonium salts; salts of organic amines, for instance salts of methylamine, dimethylamine, trimethylamine, triethylamine, ethylamine, 2-hydroxyethylamine, bis(2-hydroxyethyl)amine or tris(2-hydroxyethyl)amine; lysine or arginine salts. Salts chosen from sodium, potassium, magnesium, strontium, copper, manganese or zinc salts are preferably used. The sodium salt is preferentially used.

The (b) hydrophilic UV filter of the present invention is selected from a combination of at least one hydrophilic UV-A filter and at least one hydrophilic UV-B filter. In particular, the (b) hydrophilic UV filter is a combination of phenylbenzimidazole sulfonic acid and terephthalylidene dicamphor sulfonic acid.

The amount of the (b) hydrophilic UV filters in the composition is from 5 to 25% by weight, relative to the total weight of the composition.

In the invention, the amount of hydrophilic UV-A filter may be from 0.5 to 15% by weight, preferably from 1 to 10% by weight, and more preferably from 2 to 10% by weight, and the amount of hydrophilic UV-B filter may be from 0.5 to 25% by weight, preferably from 1 to 25% by weight, and more preferably from 5 to 20% by weight, relative to the total weight of the composition.

### (Water)

The composition according to the present invention comprises (c) water.

The amount of (c) water is not limited, and may be from 50 to 99% by weight, preferably from 55 to 95% by weight, and more preferably 60 to 90% by weight, relative to the total weight of the composition.

### (Emulsifier)

The composition according to the present invention comprises emulsifiers comprising at least one nonionic emulsifier. It may further comprise at least one emulsifier chosen from amphoteric, anionic, or cationic emulsifiers, used alone or as a mixture.

Examples of nonionic emulsifiers usable in the compositions of the invention include polyethoxylated fatty alcohols or polyglycerolated fatty alcohols, such as the adducts of ethylene oxide with lauryl alcohol, especially those containing from 9 to 50 oxyethylene units (Laureth-9 to Laureth-50 as the INCI names), in particular Laureth-9, such as the product marketed under the name "NIKKOL BL-9EX" by Nikko Chemicals Co.; esters of polyols and of a fatty acid possessing a saturated or unsaturated chain comprising, for example, from 8 to 24 carbon atoms, and their oxyalkylenated derivatives, that is to say comprising oxyethylene and/or oxypropylene units, such as esters of glycerol and of a C₈₋C₂₄ fatty acid, and their oxyalkylenated derivatives, in particular polyoxyethylenated glyceryl stearate (mono-, di- and/or tristearate), for examples PEG-20 glyceryl triisostearate ; esters of sugar and of a C₈₋C₂₄ fatty acid and their oxyalkylenated derivatives, such as polyethoxylated sorbitol esters of C₈-C₂₄ fatty acids, in particular Polysorbate 80, such as the product marketed under the name "TWEEN 80" by Croda; ethers of a sugar and of C₈₋C₂₄ fatty alcohols, such as caprylyl/capryl glucoside such as the product marketed under the name "ORAMIX CG 110" by SEPPIC; polyoxyethylene alkyl ethers; polyoxyethylene oxypropylene alkyl ethers; fatty acid alkanol amides; alkyl amine oxides; alkyl polyglycosides,and salts thereof.

Examples of anionic emulsifiers usable in the compositions of the invention include alkyl sulfates, alkyl ether sulfates, alkyl sulfonates, alkyl ether sulfonates, sulfate esters of an alkylphenoxy polyoxyethylene ethanol, alpha-olefin sulfonates, beta alkyloxy alkene sulfonates, alkyl arylsulfonates, alkyl carbonates, succinamates, sulfosuccinates, sarcosinates, octoxynol or nonoxynol phosphates, taurates, fatty taurides, sulfated monoglycerides, fatty acid amino polyoxyethylene sulfates, isethionates, alkyl benzene sulfonic acids, polyoxyethylene alkyl ether sulfuric acids, polyoxyethylene alkyl ether carboxylic acids, and polyoxyethylene alkyl amide ether carboxylic acids, and salts thereof.

Examples of amphoteric emulsifiers usable in the compositions of the invention include alkanoyl amide propyl-N,N-dimethyl glycine betaines, alkanoyl amide propyl-N,N-dimethyl-2-hydroxypropyl sulfobetaines, alkyl-N,N-dimethyl glycine betaines, alkanoyl amide propyl-N,N-dimethyl-propyl sulfobetaines, lauryl-N,N-dimethyl-2-hydroxypropyl sulfobetaines, and salts thereof.

Examples of cationic emulsifiers usable in the compositions of the invention include C₈₋C₂₄ long-chain di-alkyl dimethyl ammonium salts, long-chain mono-alkyl monobenzyl dimethyl ammonium salts and long-chain mono-alkyl trimethyl ammonium salts, all of which may have amide or ester linkages therein, and the counter ions are preferably halogen atoms such as chlorine and bromine atoms, sulfates, and alkyl group-containing sulfate residues such as methyl- and ethyl- sulfuric acid, and salts thereof. Cationic surfactants of amine type include long-chain di-alkyl monomethyl amine salts with a long-chain C₈₋C₂₄ alkyl group which may have an amide or ester linkage therein, preferably in the form of hydrochlorides, sulfates or phosphates, and salts thereof.

The salts above preferably include alkali or alkali earth metal salts, such as potassium, sodium, and magnesium salts, as well as alkanol amine salts.

The (d) emulsifier preferably comprises an anionic emulsifier, preferably selected from polyoxyethylene alkyl ether carboxylic acids, such as Laureth-3 to Laureth-17 Carboxylic Acid, Oleth-3 to Oleth-10 Carboxylic Acid referred to by their INCI names, in particular Laureth-5 Carboxylic Acid, such as the product marketed under the name "Akypo RLM 45 CA" by Kao Chemical Co. The emulsifier also preferably comprises an nonionic emulsifier, preferably selected from ethers of a sugar and of C₈₋C₂₄ fatty alcohols, such as caprylyl/capryl glucoside such as the product marketed under the name "ORAMIX CG 110" by SEPPIC.

The amount of the (d) emulsifier(s) in the composition may be from 0.1 to 30% by weight, preferably from 0.5 to 20% by weight, and more preferably from 1 to 10% by weight, relative to the total weight of the composition.

### (Other Ingredients)

The composition according to the present invention may also comprise at least one additional ingredient.

The composition according to the present invention may comprise at least one hydrophobic UV filter in addition to (b) the hydrophilic UV filter. Said hydrophobic UV filter(s) are selected from the hydrophobic UV-A filters, the hydrophobic UV-B filters and the hydrophobic UV-A and UV-B filters mentioned below.

The hydrophobic UV-A filter usable in the present invention may include aminobenzophenone compounds, such as n-hexyl 2-(4-diethlamino-2-hydroxybenzoyl)benzoate; dibenzoylmethane compounds, such as 4-isopropyldibenzoylmethane, sold under the name of "Eusolex 8020" from Merck, and butyl methoxydibenzoylmethane, sold under the trade name "Eusolex 9020" from Merck; anthranilic acid compounds, such as menthyl anthranilate marketed under the name "NEO HELIPAN MA" by Symrisc; and 4,4-diarylbutadiene compounds, such as 1,1 -dicarboxy (2,2'-dimethylpropyl)-4,4-diphenylbutadiene and diphenyl butadinene malonates and malononitriles.

The hydrophobic UV-B filter usable in the present invention may include triazine compounds, such as ethylhexyl triazone, marketed under the name "UVINUL T-150" by BASF; ethyl PABA (para-aminobenzoate); homosalate, marketed under the name "Eusolex HMS" by RonalEM industries; cinnamate compounds, such as ethylhexyl methoxycinnamate, marketed under the name "UVINUL MC 80" by BASF; β,β-diphenylacrylate compounds, such as octocrylene, marketed under the name "UVINUL N539" by BASF; benzylidenecamphor compounds, such as 3-benzylidene camphor, marketed under the name "MEXORYL SD" from CHIMEX; phenylbenzimidazole compounds, such as phenylbenzimidazolesulfonic acid, marketed under the name "Eusolex 232" by Merck; imidazoline compounds, such as ethylhexyl dimetoxybenzylidene dioxoimidazoline propionate; benzalmalonate compounds, such as polyorganosiloxane containing a benzalmalonate moiety, for example, Polysilicone-15, marketed under the name "Parsol SLX" by DSM NUTRITIONAL PRODUCTS; and merocyanine compounds.

The hydrophobic UV-A and UV-B filter usable in the present invention may include benzophenone compounds, such as benzophenone-1 marketed underthe name "UVINUL 400" by BASF; benzotriazole compounds such as drometrizole trisiloxane marketed under the name "Mexoryl XL" by BASF; bis-resorcinyl triazines, such as bis-ethylhexyloxyphenyl methoxyphenyl triazine marketed under the name "TINOSORB S" by CIBA-GEIGY; and benzoxazole compounds, such as 2,4-bis[5-(1-dimethylpropyl) benzoxazol-2-yl (4-phenyl)imino]-6-(2-ethylhexyl) imino-1,3,5-triazine marketed under the name "Uvasorb K2A" by Sigma 3V.

The amount of the hydrophobic UV filter(s) in the composition may be from 0.01 to 20% by weight, preferably from 0.1 to 10% by weight, and more preferably from 0.2 to 5% by weight, relative to the total weight of the composition.

The composition according to the present invention may comprise at least one cosmetically acceptable hydrophilic organic solvent. The hydrophilic solvent usable in the composition according to the present invention may include, for example, substantially linear or branched lower mono-alcohols having from 1 to 8 carbon atoms, such as ethanol, propanol, butanol, isopropanol, and isobutanol; aromatic alcohols, such asbenzyl alcohol and phenylethyl alcohol; polyols or polyol ethers, such as propylene glycol, dipropylene glycol, isoprene glycol, butylene glycol, glycerol, sorbitol, ethylene glycol monomethyl, monoethyl and monobutyl ethers, propylene glycol ethers, such as propylene glycol monomethylether, diethylene glycol alkyl ethers, such as diethylene glycol monoethylether or monobutylether; polyethylene glycols and their derivatives.

The amount of the hydrophilic organic solvent(s) in the composition may be from 0.1 to 40% by weight, preferably from 1 to 30% by weight, and more preferably from 2 to 25% by weight, relative to the total weight of the composition.

The composition according to the present invention may comprise at least one gelling agent. The gelling agent usable in the composition according to the present invention may include water soluble polymers such as, for example, high molecular weight crosslinked homopolymers of acrylic acid, and Aerylates/C₁₀-₃₀ Alkyl Acrylate Crosspolymer, such as the Carbopol^{®} and Pemulen^{®}; anionic acrylate polymers such as Salcare^{®} AST and cationic acrylate polymers such as Salcare^{®} SC96; acrylamidopropyltrimonium chloride/acrylamide; hydroxyethyl methacrylate polymers, Steareth-10 Allyl Ether/Acrylate Copolymer; Acrylates/Beheneth-25 Metacrylate Copolymer, known as Aculyn^{®} 28; glyceryl polymethacrylate, Acrylates/Steareth-20 Methacrylate Copolymer; bentonite; gums such as alginates, carageenans, gum acacia, gum arabic, gum ghatti, gum karaya, gum tragacanth, guar gum; guar hydroxypropyltrimonium chloride, xanthan gum or gellan gum; cellulose derivatives such as sodium carboxymethyl cellulose, hydroxyethyl cellulose, hydroxymethyl carboxyethyl cellulose, hydroxymethyl carboxypropyl cellulose, ethyl cellulose, sulfated cellulose, hydroxypropyl cellulose, methyl cellulose, hydroxypropylmethyl cellulose, microcrystalline cellulose; agar; pectin; gelatin; starch and its derivatives; chitosan and its derivatives such as hydroxyethyl chitosan; polyvinyl alcohol, PVM/MA copolymer, PVM/MA decadiene crosspolymer, polyethylene oxide) based gelling agents, sodium carbomer, and mixtures thereof.

The amount of the gelling agent(s) in the composition may be from 0.1 to 20% by weight, preferably from 0.2 to 15% by weight, and more preferably from 1 to 10% by weight, relative to the total weight of the composition.

The composition according to present invention may comprise other additives usually used in cosmetics. The additives may be selected from the group consisting of anionic, cationic, nonionic or amphoteric polymers; anionic, cationic or amphoteric surfactants; peptides and derivatives thereof; protein hydrolyzates; swelling agents and penetrating agents; natural or synthetic thickeners for (a) oils; neutralizer, such as triethanolamine; basifying agents, such as ethanol amine; acidifying agents; inorganic or organic fillers; antioxidants; preservatives; bactericides; suspending agents; sequestering agents; opacifying agents; dyes; inorganic UV filters; insoluble UV filters; vitamins or provitamins; moisturizing agents; self-tanning compounds; antiwrinkle active principles; fragrances; preserving agents, stabilizers; and mixtures thereof.

The amount of the additional ingredient(s) is not limited, but may be from 0.1 to 30% by weight relative to the total weight of the composition according to the present invention.

The composition of the present invention is in the form of an O/W emulsion having nano-sized oil droplets as the oil phase. The composition may be a cosmetic composition, preferably a cosmetic composition for a keratin substance, and more preferably a skin cosmetic composition. The composition can be in the form of a lotion, a milky lotion, a cream, a paste, a serum, or a foam, preferably a transparent or translucent composition.

The composition preferably exhibits a pH which respects the skin and which generally ranges from 3 to 8 and preferably from 4.5 to 7.

The viscosity of the composition according to the present invention is not particularly limited. The viscosity can be measured at 25°C with viscosimeters or rheometers preferably with cone-plate or plate-plate geometry. Preferably, the viscosity of the composition can range, for example, from 3 to 5000 Pa.s at 25°C and 21s⁻¹. In addition, the composition possesses a Newtonian nature.

The composition according to the present invention is preferably transparent or translucent. The transparency of the composition can be determined by measuring the turbidity with, for example, a trubidimeter (2100Q portable, Hach Company). Preferably, the composition has the turbidity more than 0, preferably more than 10, and less than 200, preferably less than 150.

The composition according to the present invention can be manufactured by mixing the components (a) to (d) and other ingredients together, for example, at room temperature (25°C).

Once preparing a dense premix composition, which is an isotropic solution, comprising at least oil, water, and emulsifier, and then diluting it to prepare the composition may be possible. In this case, the dilution factor can be varied from 5 to 40.

### [Cosmetic Use]

The composition according to the present invention may preferably be used as a cosmetic composition, preferably as a sun care cosmetic composition. In particular, the composition according to the present invention may be intended for application onto a keratin substance such as skin, scalp and/or lips, preferably the skin. Thus, the composition according to the present invention can be used for a cosmetic process for the skin. The compositions according to the present invention can further constitute a composition intended for absorbing ultraviolet light, and/or for protecting a keratin substance especially of human from ultraviolet radiation. It is well known in the art that protection of the keratin substance from ultraviolet radiation results in anti-ageing, anti-wrinkle, and moisturizing. Accordingly, the composition of the present invention can further constitute a composition intended for anti-aging, anti-wrinkle and/or moisturizing.

A further subject-matter of the invention is a composition according to the invention for use in a process for protecting a keratin substance such as skin, from ultraviolet radiation, which is applied onto the keratin substance. The present invention can also relates to a method of protecting a keratin substance from ultraviolet radiation comprising applying to the keratin substance the composition according to the present invention, as well as a method of absorbing ultraviolet light comprising applying the composition according to the present invention and subjecting the keratin substance to ultraviolet light. These methods can be defined as non-therapeutic methods.

The composition according to the present invention can be used in the topical sun care composition in the form of a lotion, a milky lotion, a cream, a gel, a paste, a serum, a foam, or a spray, preferably be in the form of a gel or a spray, and more preferably a transparent or translucent gel.

### EXAMPLES

The present invention will be described in a more detailed manner by way of examples. However, these examples should not be construed as limiting the scope of the present invention.

### [Examples 1-2 and Comparative Examples 1-2]

### (Preparation of the Compositions according to Examples 1 and 2)

Premix Compositions 1 and 2 were prepared by gently mixing the ingredients with an overhead stirrer at room temperature. The ingredients and the compositions thereof are shown below. The numerical values for the amounts of the ingredients are all based on "% by weight" as active raw materials.

| Ingredients | Premix Composition 1 | Premix Composition 2 |
|---|---|---|
| Laureth 5 carboxylic acid | 20 | 20 |
| Caprylyl/capryl glucoside | 10 | 10 |
| Ethyl hexyl methoxycinnamate | 10 | - |
| Ethyl hexyl palmitate | 20 | 20 |
| Water | 40 | 50 |

The compositions according to Examples 1 and 2 were prepared by gently mixing Premix Compositions 1 and 2 obtained above, respectively, with water containing phenylbenzimidazole sulfonic acid and terephthalylidene dicamphor sulfonic acid as hydrophilic UV filters and triethanolamine as a neutralizer at room temperature. The obtained compositions are summarized in Table 1 below. The numerical values for the amounts of the ingredients shown in Table 1 are all based on "% by weight" as active raw materials.

### (Preparation of the Composition according to Comparative Example 1)

The composition according to Comparative Example 1 was prepared in the same way as Examples 1 and 2 above, except that Premix Composition 3 below is used. This composition did not include oil, and thus consisted of only an aqueous phase.

| Ingredients | Premix Composition 3 | |
|---|---|---|
| Laureth 5 carboxylic acid | | 20 |
| Caprylyl/capryl glucoside | | 10 |
| Ethyl hexyl methoxycinnamate | | - |
| Ethyl hexyl palmitate | | - |
| Water | | 60 |

### (Preparation of the Composition according to Comparative Example 2)

The composition according to Comparative Example 2, shown in Table 1, was prepared by vigorously mixing the ingredients with a magnetic stirrer at 2700 rpm at 25 °C for 10 min.

**Table 1**

| | Ex. 1 | Ex. 2 | Comp. Ex. 1 | Comp. Ex. 2 |
|---|---|---|---|---|
| Laureth 5 carboxylic acid | 2.0 | 2.0 | 2.0 | 2.0 |
| Caprylyl/capryl glucoside | 1.0 | 1.0 | 1.0 | 1.0 |
| Ethyl hexyl methoxycinnamate | - | 1.0 | - | 1.0 |
| Ethyl hexyl palmitate | 2.0 | 2.0 | - | 2.0 |
| Water | 74.2 | 73.2 | 76.2 | 73.2 |
| Phenylbenzimidazole sulfonic acid | 3.5 | 3.5 | 3.5 | 3.5 |
| Terephthalylidene dicamphor sulfonic acid | 13.3 | 13.3 | 13.3 | 13.3 |
| Triethanolamine | 4 | 4 | 4 | 4 |

### [Evaluation]

The compositions according to Examples 1-2 and Comparative Examples 1-2 were evaluated as follows.

### (Droplet size)

Particle size analyzer (Vasco, Cordouan Technologies) was used to determine the droplet size of the oil phase of the O/W emulsion compositions according to Examples 1-2 and Comparative Example 2. The refractive index and viscosity of the continuous aqueous phase were 1.33 and 0.89 cp at 25 °C.

### (Turbidity)

The turbidities of the compositions according to Examples 1-2 and Comparative Examples 1-2 were measured at room temperature by using a trubidimeter (2100Q portable, Hach Company). The meaning of the turbidity value (NTU) was defined as follows:

### NTU

| | |
|---|---|
| 1-50: | Transparent |
| 50-150: | Translucent |
| >200: | Turbid or Opaque |

The turbidity of the composition according to Comparative Example 2 was not measurable due to its too high turbidity (>1000 NTU).

### (SPF)

The sample for SPF value measurement was prepared by placing 20 mg of each of the compositions according to Examples 1-2 and Comparative Examples 1-2 on the plate (Helio plate HD 6, PMMA, 50 mm × 50 mm) like dots, then spreading them 3 times with a finger over the plate. The in vitro SPF value was calculated from the UV absorbance data, which was measured with a UV spectrophotometer (UV-2000S, Labsphere Inc) on the diffuse transmittance of the samples in the wavelength range from 250 to 450 nm.

The results of these evaluations are shown in Table 2.

**Table 2**

| | Ex. 1 | Ex. 2 | Comp. Ex. 1 | Comp. Ex. 2 |
|---|---|---|---|---|
| Oil Droplet size (nm) | 100 | 100 | - | 10000 |
| Turbidity (NTU) | 90 | 110 | 1 | N.A. |
| SPF value | 10 | 11 | 7 | 8 |

| | | | | |
|---|---|---|---|---|
| N.A. = not available | | | | |

The compositions according to Examples 1 and 2 were able to provide better UV protecting effect. Furthermore, the appearances of these compositions were translucent with low turbidities, which are preferable in use for skin cosmetic compositions.

On the other hand, the compositions according to Comparative Example 1, which was a homogeneous composition consisting of water phase and not including oil phase, exhibited poor UV protecting effect, even though it includes the same amount of hydrophilic UV filter as the compositions according to Examples 1 and 2. In addition, the composition according to Comparative Example 2, which includes the oil phase droplets having the number-average diameter of 10000 nm, also exhibited poor UV protecting effect. Furthermore, the composition according to Comparative Example 2 has such a high turbidity that it cannot be measured with the trubidimeter.

Thus, it is clear that only the compositions according to the present invention can produce improved UV protecting effect as an O/W emulsion.

## Claims

1. A composition in the form of an O/W emulsion having oil phases dispersed in an aqueous phase, comprising:
(a) at least one oil;
(b) at least two hydrophilic UV filters comprising at least one hydrophilic UV-A filter and at least one hydrophilic UV-B filter;
(c) water; and
(d) emulsifiers comprising at least one nonionic emulsifier;
wherein
the number-average diameter of the oil phase droplets is less than 200 nm, and the amount of the oil is from 0.5 to 5% by weight relative to the total weight of the composition;
the hydrophilic UV-A filter is selected from terephthalylidene dicamphor sulfonic acid and salts thereof, bisbenzoxazolyl derivatives chosen from disodium phenyl dibenzimidazole tetrasulfonate and salts thereof;
the hydrophilic UV-B filter is selected from p-aminobenzoic (PABA) derivatives chosen from PABA, Glyceryl PABA and PEG-25 PABA and salts thereof, phenylbenzimidazole sulfonic acid and salts thereof, ferulic acid and salts thereof, salicylic acid and salts thereof, DEA methoxycinnamate and salts thereof, benzylidene camphor sulfonic acid and salts thereof, and camphor benzalkonium methosulfate and salts thereof; and
the amount of the (b) hydrophilic UV filters is from 5 to 25% by weight relative to the total weight of the composition.

2. The composition according to Claim 1, wherein the number-average diameter of the oil phase droplets is less than 180 nm, preferably less than 160 nm, and more preferably less than 140 nm.

3. The composition according to Claim 1 or 2, wherein the number-average diameter of the oil phase droplets is more than 1 nm, more preferably more than 5 nm, and even more preferably more than 10 nm, and in particular more than 20 nm.

4. The composition according to any one of Claims 1 to 3, wherein the oil is selected from ester oils having a molecular weight less than 600 g/mol such as isopropyl myristate, isopropyl palmitate, ethyl hexyl palmitate, artificial triglycerides such as capryl caprylyl glycerides; hydrocarbon oil; and mineral oil such as paraffine oil and mixtures thereof.

5. The composition according to any one of Claims 1 to 4, wherein the amount of the oil is from 1 to 5 % by weight, relative to the total weight of the composition.

6. The composition according to any one of Claims 1 to 5, wherein the emulsifiers comprise an anionic emulsifier.

7. The composition according to any one of Claims 1 to 6, wherein the hydrophilic UV filter is selected from phenylbenzimidazole sulfonic acid, terephthalylidene dicamphor sulfonic acid, and mixtures thereof.

8. The composition according to any one of Claims 1 to 7, wherein the amount of the water in the composition is from 50 to 99% by weight, preferably from 55 to 95% by weight, and more preferably 60 to 90% by weight, relative to the total weight of the composition.

9. The composition according to any one of Claims 1 to 8, wherein the composition comprises at least one hydrophobic UV filter, preferably in an amount of from 0.1 to 10% by weight, and more preferably from 0.2 to 5% by weight, relative to the total weight of the composition
wherein the hydrophobic UV filter is selected from aminobenzophenone compounds, dibenzoylmethane compounds, anthranilic acid compounds, 4,4-diarylbutadiene compounds, malonitriles, triazine compounds, ethyl para-aminobenzoate, homosalate, cinnamate compounds, β,β-diphenylacrylate compounds, benzylidenecamphor compounds, phenylbenzimidazolesulfonic acid, imidazoline compounds, benzalmalonate compounds, merocyanine compounds, benzophenone compounds, benzotriazole compounds, bis-resorcinyl triazines, and benzoxazole compounds.

10. The composition according to any one of Claims 1 to 9, wherein the composition further comprises at least one gelling agent, preferably in an amount of from 0.1 to 20% by weight, preferably from 0.2 to 15% by weight, and more preferably from 1 to 10% by weight, relative to the total weight of the composition.

11. The composition according to any one of Claims 1 to 10, wherein the nonionic emulsifier is selected from ethers of a sugar and of C₈₋C₂₄ fatty alcohols.

12. The composition according to any one of Claims 1 to 11, wherein the amount of the emulsifier is from 0.1 to 30% by weight, preferably from 0.5 to 20% by weight, and more preferably from 1 to 10% by weight, relative to the total weight of the composition.

13. The composition according to any one of Claims 1 to 12, wherein the composition is a cosmetic composition, preferably a cosmetic composition for a keratin substance, and more preferably a skin cosmetic composition.

14. Composition according to any one of Claims 1 to 13, for use in a process for protecting a keratin substance from ultraviolet radiation, which is applied onto the keratin substance.

## Patentansprüche

1. Zusammensetzung in Form einer Ö/W-Emulsion mit Ölphasen, die in einer wässrigen Phase dispergiert sind, umfassend:
(a) mindestens ein Öl;
(b) mindestens zwei hydrophile UV-Filter, die mindestens einen hydrophilen UV-A-Filter und mindestens einen hydrophilen UV-B-Filter umfassen;
(c) Wasser; und
(d) Emulgatoren, die mindestens einen nichtionischen Emulgator umfassen;
wobei
der zahlengemittelte Durchmesser der Ölphasentröpfchen kleiner ist als 200 nm, und die Menge des Öls von 0,5 bis 5 Gew.-% beträgt, bezogen auf das Gesamtgewicht der Zusammensetzung;
der hydrophile UV-A-Filter ausgewählt ist aus Terephthalylidendikampfersulfonsäure und deren Salzen, Bisbenzoxazolylderivaten, die aus Dinatriumphenyldibenzimidazoltetrasulfonat und dessen Salzen ausgewählt sind;
der hydrophile UV-B-Filter aus p-Aminobenzoesäure (PABA)-Derivaten ausgewählt ist, die aus PABA, Glyceryl-PABA und PEG-25-PABA und deren Salzen, Phenylbenzimidazolsulfonsäure und deren Salzen, Ferulasäure und deren Salzen, Salicylsäure und deren Salzen, DEA-Methoxycinnamat und dessen Salzen, Benzylidenkampfersulfonsäure und deren Salzen, und Kampferbenzalkoniummethosulfat und dessen Salzen ausgewählt sind; und die Menge der (b) hydrophilen UV-Filter von 5 bis 25 Gew.-% beträgt, bezogen auf das Gesamtgewicht der Zusammensetzung.

2. Zusammensetzung nach Anspruch 1, wobei der zahlengemittelte Durchmesser der Ölphasentröpfchen kleiner ist als 180 nm, vorzugsweise kleiner als 160 nm und mehr bevorzugt kleiner als 140 nm.

3. Zusammensetzung nach Anspruch 1 oder 2, wobei der zahlengemittelte Durchmesser der Ölphasentröpfchen größer ist als 1 nm, mehr bevorzugt größer als 5 nm, und noch mehr bevorzugt größer als 10 nm, und insbesondere größer als 20 nm.

4. Zusammensetzung nach einem der Ansprüche 1 bis 3, wobei das Öl ausgewählt ist aus Esterölen mit einem Molekulargewicht kleiner als 600 g/mol wie zum Beispiel Isopropylmyristat, Isopropylpalmitat, Ethylhexylpalmitat, künstliche Triglyceride wie zum Beispiel Caprylcaprylylglyceride; Kohlenwasserstofföl; und Mineralöl wie zum Beispiel Paraffinöl und Mischungen davon.

5. Zusammensetzung nach einem der Ansprüche 1 bis 4, wobei die Menge des Öls von 1 bis 5 Gew.-% beträgt, bezogen auf das Gesamtgewicht der Zusammensetzung.

6. Zusammensetzung nach einem der Ansprüche 1 bis 5, wobei die Emulgatoren einen anionischen Emulgator umfassen.

7. Zusammensetzung nach einem der Ansprüche 1 bis 6, wobei der hydrophile UV-Filter ausgewählt ist aus Phenylbenzimidazolsulfonsäure, Terephthalylidendikampfersulfonsäure und Mischungen davon.

8. Zusammensetzung nach einem der Ansprüche 1 bis 7, wobei die Menge an Wasser in der Zusammensetzung von 50 bis 99 Gew.-%, vorzugsweise von 55 bis 95 Gew.-% und mehr bevorzugt von 60 bis 90 Gew.-% beträgt, bezogen auf das Gesamtgewicht der Zusammensetzung.

9. Zusammensetzung nach einem der Ansprüche 1 bis 8, wobei die Zusammensetzung mindestens einen hydrophoben UV-Filter umfasst, vorzugsweise in einer Menge von 0,1 bis 10 Gew.-% und mehr bevorzugt von 0,2 bis 5 Gew.-%, bezogen auf das Gesamtgewicht der Zusammensetzung, wobei der hydrophobe UV-Filter ausgewählt ist aus Aminobenzophenonverbindungen, Dibenzoylmethanverbindungen, Anthranilsäureverbindungen, 4,4-Diarylbutadienverbindungen, Malonitrilen, Triazinverbindungen, Ethyl-para-aminobenzoat, Homosalat, Cinnamatverbindungen, β,β-Diphenylacrylatverbindungen, Benzylidenkampferverbindungen, Phenylbenzimidazolsulfonsäure, Imidazolinverbindungen, Benzalmalonatverbindungen, Merocyaninverbindungen, Benzophenonverbindungen, Benzotriazolverbindungen, bis-Resorcinyltriazinen und Benzoxazolverbindungen.

10. Zusammensetzung nach einem der Ansprüche 1 bis 9, wobei die Zusammensetzung ferner mindestens ein Geliermittel umfasst, vorzugsweise in einer Menge von 0,1 bis 20 Gew.-%, vorzugsweise von 0,2 bis 15 Gew.-% und mehr bevorzugt von 1 bis 10 Gew.-%, bezogen auf das Gesamtgewicht der Zusammensetzung.

11. Zusammensetzung nach einem der Ansprüche 1 bis 10 wobei der nichtionische Emulgator aus Ethern eines Zuckers und aus C₈-C₂₄-Fettalkoholen ausgewählt ist.

12. Zusammensetzung nach einem der Ansprüche 1 bis 11, wobei die Menge des Emulgators von 0,1 bis 30 Gew.-%, vorzugsweise von 0,5 bis 20 Gew.-% und mehr bevorzugt von 1 bis 10 Gew.-% beträgt, bezogen auf das Gesamtgewicht der Zusammensetzung.

13. Zusammensetzung nach einem der Ansprüche 1 bis 12, wobei die Zusammensetzung eine kosmetische Zusammensetzung ist, vorzugsweise eine kosmetische Zusammensetzung für eine Keratinsubstanz, und mehr bevorzugt eine hautkosmetische Zusammensetzung.

14. Zusammensetzung nach einem der Ansprüche 1 bis 13, zur Verwendung bei einem Verfahren zum Schutz einer Keratinsubstanz vor ultravioletter Strahlung, die auf die Keratinsubstanz aufgebracht wird.

## Revendications

1. Composition sous la forme d'une émulsion huile dans l'eau ayant des phases huileuses dispersées dans une phase aqueuse, comprenant :
(a) au moins une huile ;
(b) au moins deux filtres UV hydrophiles comprenant au moins un filtre UV-A hydrophile et au moins un filtre UV-B hydrophile ;
(c) de l'eau ; et
(d) des émulsifiants comprenant au moins un émulsifiant non ionique ;
dans lequel
le diamètre moyen en nombre des gouttelettes de phase huileuse est inférieur à 200 nm, et la quantité de l'huile est de 0,5 % à 5 % en poids par rapport au poids total de la composition ;
le filtre UV-A hydrophile est sélectionné parmi l'acide téréphtalylidène dicamphre sulfonique et des sels de celui-ci, des dérivés de bisbenzoxazolyle choisis parmi le tétrasulfonate de disodium phényl dibenzimidazole et des sels de celui-ci ;
le filtre UV-B hydrophile est sélectionné parmi des dérivés de l'acide p-aminobenzoïque (PABA) choisis parmi le PABA, le glycéryl PABA et le PEG-25 PABA et des sels de ceux-ci, l'acide phénylbenzimidazole sulfonique et des sels de celui-ci, l'acide férulique et des sels de celui-ci, l'acide salicylique et des sels de celui-ci, le DEA méthoxycinnamate et des sels de celui-ci, l'acide benzylidène camphre sulfonique et des sels de celui-ci, et le méthosulfate de benzalkonium de camphre et des sels de celui-ci ; et
la quantité des filtres UV hydrophiles (b) est de 5 à 25 % en poids par rapport au poids total de la composition.

2. Composition selon la revendication 1, dans laquelle le diamètre moyen en nombre des gouttelettes de phase huileuse est inférieur à 180 nm, de préférence inférieur à 160 nm et de manière davantage préférée inférieur à 140 nm.

3. Composition selon la revendication 1 ou 2, dans laquelle le diamètre moyen en nombre des gouttelettes de phase huileuse est supérieur à 1 nm, de préférence supérieur à 5 nm et de manière davantage préférée supérieur à 10 nm, et en particulier supérieur à 20 nm.

4. Composition selon l'une quelconque des revendications 1 à 3, dans laquelle l'huile est sélectionnée parmi des huiles d'ester ayant un poids moléculaire inférieur à 600 g/mol telles que le myristate d'isopropyle, le palmitate d'isopropyle, le palmitate d'éthyle hexyle, des triglycérides artificiels tels que des glycérides de capryle-caprylyle ; une huile hydrocarbonée ; et une huile minérale telle que l'huile de paraffine et des mélanges de celles-ci.

5. Composition selon l'une quelconque des revendications 1 à 4, dans laquelle la quantité de l'huile est de 1 à 5 % en poids, par rapport au poids total de la composition.

6. Composition selon l'une quelconque des revendications 1 à 5, dans laquelle les émulsifiants comprennent un émulsifiant anionique.

7. Composition selon l'une quelconque des revendications 1 à 6, dans laquelle le filtre UV hydrophile est sélectionné parmi l'acide phénylbenzimidazole sulfonique, l'acide téréphtalylidène dicamphre sulfonique et des mélanges de ceux-ci.

8. Composition selon l'une quelconque des revendications 1 à 7, dans laquelle la quantité de l'eau dans la composition est de 50 à 99 % en poids, de préférence de 55 à 95 % en poids et de manière davantage préférée de 60 à 90 % en poids, par rapport au poids total de la composition.

9. Composition selon l'une quelconque des revendications 1 à 8, dans laquelle la composition comprend au moins un filtre UV hydrophobe, de préférence en une quantité de 0,1 à 10 % en poids et de manière davantage préférée de 0,2 à 5 % en poids, par rapport au poids total de la composition
dans laquelle le filtre UV hydrophobe est sélectionné parmi des composés d'aminobenzophénone, des composés de dibenzoylméthane, des composés d'acide anthranilique, des composés de 4,4-diarylbutadiène, des malonitriles, des composés de triazine, des composés de para-aminobenzoate d'éthyle, l'homosalate, des composés de cinnamate, des composés de β-β-diphénylacrylate, des composés de benzylidènecamphre, l'acide phénylbenzimidazole sulfonique, des composés d'imidazoline, des composés de benzalmalonate, des composés de mérocyanine, des composés de benzophénone, des composés de benzotriazole, des bis-résorcinyl triazines et des composés de benzoxazole.

10. Composition selon l'une quelconque des revendications 1 à 9, dans laquelle la composition comprend en outre au moins un agent gélifiant, de préférence en une quantité de 0,1 à 20 % en poids, de préférence de 0,2 à 15 % en poids et de manière davantage préférée de 1 à 10 % en poids, par rapport au poids total de la composition.

11. Composition selon l'une quelconque des revendications 1 à 10, dans laquelle l'émulsifiant non ionique est sélectionné parmi des éthers d'un sucre et d'alcools gras en C₈-C₂₄.

12. Composition selon l'une quelconque des revendications 1 à 11, dans laquelle la quantité de l'émulsifiant est de 0,1 à 30 % en poids, de préférence de 0,5 à 20 % en poids et de manière davantage préférée de 1 à 10 % en poids, par rapport au poids total de la composition.

13. Composition selon l'une quelconque des revendications 1 à 12, dans laquelle la composition est une composition cosmétique, de préférence une composition cosmétique pour une substance kératinique et de manière davantage préférée une composition cosmétique pour la peau.

14. Composition selon l'une quelconque des revendications 1 à 13, pour une utilisation dans un processus de protection d'une substance kératinique contre le rayonnement ultraviolet, qui est appliquée sur la substance kératinique.
